Europäisches Patentamt

European Patent Office

Office européen des brevets

(11) Veröffentlichungsnummer: **0 085 376**
**A2**

## (12) EUROPÄISCHE PATENTANMELDUNG

(21) Anmeldenummer: **83100583.0**

(22) Anmeldetag: **24.01.83**

(51) Int. Cl.³: **B 01 J 2/00**
**A 01 N 25/12, A 61 K 9/46**
**C 09 B 67/00**

(30) Priorität: **02.02.82 DE 3203443**

(43) Veröffentlichungstag der Anmeldung:
**10.08.83 Patentblatt 83/32**

(84) Benannte Vertragsstaaten:
**AT BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Hausmann, Heinz, Dr.**
**Dierath 5**
**D-5653 Leichlingen 1(DE)**

(72) Erfinder: **Neumaier, Hermann, Dr.**
**Kurlandweg 33**
**D-5653 Leichlingen 1(DE)**

(54) **Wasserdispergierbare bzw. wasserlösliche Granulate, Verfahren zu deren Herstellung und deren Verwendung.**

(57) In Wasser dispergierbare bzw. lösliche Granulate, die
- 5 bis 80 Gew.-% an mindestens einer aktiven Komponente,
- 5 bis 30 Gew.-% an mindestens einem zur Gaserzeugung befähigten Produkt,
- 2 bis 60 Gew.-% an Zusatzstoffen und gegebenenfalls
- 1 bis 20 Gew.-% an Granulierflüssigkeit

enthalten, wobei die Summe der Bestandteile jeweils 100 Gew.-% beträgt,
ein Verfahren zu deren Herstellung sowie deren Verwendung in der Landwirtschaft und im Gartenbau, im Haushalts- und Hygiene-Bereich, im medizinischen Bereich oder in der Farbstoffindustrie.

EP 0 085 376 A2

0085376

BAYER AKTIENGESELLSCHAFT     5090 Leverkusen, Bayerwerk

Zentralbereich               Dü/bc/c
Patente, Marken und Lizenzen  V


**Wasserdispergierbare bzw. wasserlösliche Granulate,
Verfahren zu deren Herstellung und deren Verwendung**

Die vorliegende Erfindung betrifft neue wasserdispergierbare bzw. wasserlösliche Granulate, ein Verfahren zu deren Herstellung und deren Verwendung.

Es ist bereits bekannt, daß wasserdispergierbare Granulate durch mischende und rollende Bewegung des zu granulierenden Materials in entsprechenden Apparaturen unter gleichzeitiger Zugabe geringer Mengen an Flüssigkeit (Wasser bzw. Wasser und organische Lösungsmittel) gebildet werden (vgl. H.B. Ries "Aufbaugranulierung" in Aufbereitungstechnik 11, (1971), Seiten 675 ff).

Weiterhin ist es schon bekannt, wasserdispergierbare Granulate herzustellen, indem man dem zu granulierenden Material in einer Granuliertrommel soviel Granulierflüssigkeit zugibt, daß die Gleichgewichtsfeuchte dieses Materials überschritten wird, und man anschließend das erhaltene Produkt wieder bis auf oder unter die Gleichgewichtsfeuchte heruntertrocknet (vgl.

Le A 21 434 -Ausland

DE-OS 2 723 221).

Außerdem wurde bereits die Herstellung von wasserdispergierbaren Granulaten nach dem Wirbelschichtverfahren beschrieben (vgl. US-PS 3 920 442). Hierbei wird das fein zerteilte Material mit einer wäßrigen Lösung besprüht, die ein Bindemittel enthält.

Bekannt ist schließlich auch die Herstellung von wasserdispergierbaren bzw. wasserlöslichen Granulaten nach dem Sprühtrocknungsverfahren sowie durch Tablettierung von Pulvermischungen oder durch Pressen von Pulvermischungen.

Nachteilig an diesen bekannten Verfahren ist, daß im allgemeinen ein relativ hoher maschineller Aufwand erforderlich ist, und die Verfahren schwierig zu steuern sind, da viele Parameter bei der Herstellung zu beachten sind. Außerdem handelt es sich bei diesen Granulaten in vielen Fällen um lockere Materialien, deren Schüttgewicht gering ist und die deshalb ein verhältnismäßig großes Packvolumen besitzen. Darüber hinaus neigen derartige Granulate zur Staubbildung und lassen sich nicht immer ohne Schwierigkeiten in wäßrigem Medium dispergieren oder lösen, da ihre Dichte niedriger ist als diejenige des Wassers. Ungünstig ist auch, daß die nach diesen bekannten Verfahren hergestellten Granulate häufig von sehr unterschiedlicher Korngröße sind, also ein breites Korngrößenspektrum besitzen. Die Dispergierbarkeit bzw. Löslichkeit der nach den bekannten Press- bzw. Ta-

Le A 21 434

blettierverfahren erhältlichen Granulate bzw. Pellets
in Wasser ist ebenfalls nicht immer befriedigend.

Es wurden nun neue wasserdispergierbare bzw. wasserlösliche Granulate gefunden, die

- 5 bis 80 Gew.-% an mindestens einer aktiven Komponente,
- 5 bis 30 Gew.-% an mindestens einem zur Gaserzeugung befähigten Produkt,
- 2 bis 60 Gew.-% an Zusatzstoffen und gegebenenfalls
- 1 bis 20 Gew.-% an Granulierflüssigkeit

enthalten, wobei die Summe der Bestandteile jeweils
100 Gew.-% beträgt.

Weiterhin wurde gefunden, daß sich die erfindungsgemäßen wasserdispergierbaren bzw. wasserlöslichen Granulate dadurch herstellen lassen, daß man

- mindestens eine aktive Komponente mit mindestens
  einem zur Gaserzeugung befähigten Produkt sowie
  mit Zusatzstoffen vermengt,
- das entstehende Gemisch fein mahlt, danach mit Granulierflüssigkeit versetzt, gegebenenfalls trocknet
  und
- pelletiert oder tablettiert.

Schließlich wurde gefunden, daß sich die erfindungsgemäßen wasserdispergierbaren bzw. wasserlöslichen
Granulate je nach den enthaltenen aktiven Komponenten

Le A 21 434

für verschiedenste Zwecke in der Landwirtschaft und im Gartenbau, im Haushalts- und Hygiene-Bereich, im medizinischen Bereich oder in der Farbstoffindustrie verwenden lassen.

Es ist als äußerst überraschend zu bezeichnen, daß die erfindungsgemäßen Granulate in Wasser vollständig und schnell dispergierbar bzw. löslich sind, denn bei der relativ hohen Dichte und Kompaktheit der erfindungsgemäßen Agglomerate wäre stattdessen eine Sedimentation und Klumpenbildung in Wasser zu erwarten gewesen.

Die erfindungsgemäßen wasserdispergierbaren bzw. wasserlöslichen Granulate zeichnen sich durch eine Reihe von Vorteilen aus. So besitzen diese Granulate eine hohe Dichte und ein hohes Schüttgewicht, was zur Folge hat, daß das Packvolumen klein ist. Günstig ist auch, daß diese maschinell tablettierten oder pelletierten Granulate eine variierbare, innerhalb enger Grenzen einheitliche Korngröße aufweisen und deshalb eine Dosierung problemlos ist. Ferner sind die erfindungsgemäßen Granulate wegen ihrer mechanischen Festigkeit staubarm und auch bei allen in der Praxis vorkommenden mechanischen Beanspruchungen gegen Abrieb stabil. Das erfindungsgemäße Verfahren zur Herstellung der wasserdispergierbaren bzw. wasserlöslichen Granulate erfordert einen geringen maschinellen und personellen Aufwand und ist einfach kontrollierbar. Außerdem ist der Verbrauch an Energie vergleichsweise niedrig, da ein Trocknungsprozeß sich in vielen Fällen erübrigt. Darüber hinaus ist das erfindungsgemäße Verfahren nicht

Le A 21 434

auf die Granulierung weniger Substanzen begrenzt, sondern bezüglich der aktiven Komponenten extrem breit anwendbar. Es kann schließlich unabhängig von der Menge des zu granulierenden Materials sowohl kontinuierlich als auch diskontinuierlich durchgeführt werden und erlaubt einen raschen, komplikationslosen Produktwechsel.

Die erfindungsgemäßen Granulate können eine oder auch mehrere aktive Komponenten enthalten. Als aktive Komponenten kommen sowohl solche Substanzen in Frage, die bei Raumtemperatur fest sind, als auch solche, die bei Raumtemperatur flüssig sind. Voraussetzung für die Verwendung flüssiger aktiver Komponenten ist lediglich, daß sie vor der Granulierung auf feste Trägerstoffe aufgezogen werden. Die aktiven Komponenten können in Wasser löslich oder unlöslich sein.

Als aktive Komponenten kommen agrochemische Stoffe, Wirkstoffe zur Bekämpfung von Schädlingen im Haushalts- und Hygiene-Bereich, pharmakologisch wirksame Stoffe und Farbstoffe in Betracht.

Unter agrochemischen Stoffen sind hierbei üblicherweise im Pflanzenschutz verwendbare Wirkstoffe zu verstehen. Hierzu gehören vorzugsweise Insektizide, Akarizide, Nematizide, Fungizide, Herbizide, Wachstumsregulatoren und Düngemittel. Als Beispiele für derartige Wirkstoffe seien im einzelnen genannt:

O,O-Diethyl-O-(4-nitro-phenyl)-thiono-phosphorsäureester, O,O-Dimethyl-O-(4 itro-phenyl)-thiono-phosphor-

Le A 21 434

säureester, O-Ethyl-O-(4-methylthio-phenyl)-S-propyl-dithiophosphat, (O,O-Diethylthionophosphoryl)-$\alpha$-oximino-phenylessigsäurenitril, 2-Isopropoxy-phenyl-N-methyl-carbamat, 3-Methylthio-4-amino-6-tert.-butyl-1,2,4-triazin-5-on, 3-Methylthio-4-isobutylidenamino-6-tert.-butyl-1,2,4-triazin-5-on, 2-Chlor-4-ethylamino-6-iso-propylamino-1,3,5-triazin, 2,3-Dihydro-2,2-dimethyl-7-benzofuranyl-methyl-carbamat, 3,5-Dimethyl-4-methyl-thiophenyl-N-methyl-carbamat, O,O-Diethyl-O-(3-chlor-4-methyl-7-cumarinyl)-thiophosphat, $\gamma$-Hexachlorcyclo-hexan, 6,7,8,9,10,10-Hexachlor-1,5,5A,6,9,9A-hexahydro-6,9-methan-2,4,3-benzo-dioxathiepin-3-oxid, 1,4,5,6,7,8,8-Heptachlor-4,7-endo-methylen-3A,4,7,7A-tetrahydroinden, 2-(2-Furyl)-benzimidazol, 5-Amino-1-bis-(dimethylamido)-phosphoryl-3-phenyl-1,2,4-triazol, 4-Hydroxy-3-(1,2,3,4-tetrahydro-1-naphthyl)-cumarin, S-/1,2-Bis-(ethoxycar-bonyl)-ethyl_7-O,O-dimethyl-dithiophosphorsäureester, O,O-Dimethyl-O-(4-methylmercapto-3-methyl-phenyl)-thionophosphorsäurester, O-Ethyl-O-(2-isopropyloxy-carbonyl-phenyl)-N-isopropyl-thionophosphorsäureester-amid, 1-(4-Chlorphenoxy)-3,3-dimethyl-1-(1,2,4-triazol-1-yl)-2-butanon, (S)-$\alpha$-Cyano-3-phenoxybenzyl(1R)-cis-3-(2,2-dibromvinyl)-2,2-dimethylcyclopropancarb-oxylat.

Unter Wirkstoffen zur Bekämpfung von Schädlingen im Haushalts- und Hygiene-Bereich sind im vorliegenden Fall üblicherweise für derartige Zwecke einsetzbare Stoffe zu verstehen. Als Beispiele seien genannt:

2-Isopropoxy-phenyl-N-methylcarbamat, O,O-Diethyl-O-

Le A 21 434

(4-nitro-phenyl)-thionophosphorsäureester, O,O-Dimethyl-O-(4-nitro-phenyl)-thionophosphorsäureester, S-/1,2-Bis-(ethoxycarbonyl)-ethyl_7-O,O-dimethyl-dithiophosphorsäureester, O,O-Dimethyl-O-(3-methyl-4-nitro-phenyl)-thionophosphorsäureester, O,O-Dimethyl-O-(4-methylmercapto-3-methyl-phenyl)-thionophosphorsäureester, (Cyclohex-1-en-1,2-dicarboximidomethyl)-2,2-dimethyl-3-(2-methylpropenyl)-cyclopropancarboxylat.

Unter pharmakologisch wirksamen Stoffen sind im vorliegenden Fall vorzugsweise im veterinärmedizinischen Bereich einsetzbare Stoffe zu verstehen. Als Beispiel für derartige Wirkstoffe sei genannt:

2,2-Dimethyl-3-/ß-(p-chlorphenyl)-ß-chlorvinyl_7-cyclopropancarbonsäure-d-cyano-3-phenoxy-4-fluor-benzylester.

Unter Farbstoffen sind im vorliegenden Fall zur Herstellung von Farbstoffdispersionen bzw. Farbstofflösungen geeignete Stoffe zu verstehen, die als Färbemittel und/oder Anstrichstoffe Verwendung finden. Als Beispiele seien genannt:

Triarylaminfarbstoffe, Triarylmethanfarbstoffe, Methinfarbstoffe, Anthrachinonfarbstoffe, Indigofarbstoffe, Schwefelfarbstoffe, Azofarbstoffe und Pigmentfarbstoffe.

Als zur Gaserzeugung befähigte Produkte können in den erfindungsgemäßen Granulaten alle diejenigen Substanzen oder Substanzgemische enthalten sein, die in der

Le A 21 434

Lage sind, bei der Dispergierung bzw. beim Lösen der Granulate in Wasser Gas in Freiheit zu setzen, und die nicht mit den jeweils enthaltenen aktiven Komponenten reagieren. Zu derartigen Gasgeneratoren gehören vorzugsweise Produkte, die unter den genannten Bedingungen als Kohlendioxid- oder Stickstoff-Lieferanten fungieren können, wie z.B. Gemische aus Säure und Carbonat, Gemische aus Säureanhydrid und Carbonat, Stickstoff-abspaltende Substanzen sowie aus Bakterien und Milchsäure bestehende Gemische, die zur Kohlendioxid-Erzeugung in der Lage sind.

Als Säuren, die in den Gemischen aus Säure und Carbonat enthalten sein können, seien genannt: Gesättigte oder ungesättigte aliphatische Mono-, Di- und Tricarbonsäuren sowie Hydroxycarbonsäuren, wie Essigsäure, Propionsäure, Bernsteinsäure, Adipinsäure, Zitronensäure, Propantricarbonsäure, Maleinsäure, Fumarsäure und Aconitsäure, ferner Aminosäuren, wie Glycin, weiterhin gegebenenfalls substituierte Monosulfonsäuren, wie Di-isobutylnaphthalinsulfonsäure und n-Dodecylbenzolsulfonsäure, außerdem gegebenenfalls substituierte aromatische Carbonsäuren, wie Benzoesäure, Terephthalsäure, Gallensäuren und Harzsäure, und schließlich von Heterocyclen abgeleitete Carbonsäuren, wie Nucleinsäuren.

Als Carbonsäureanhydride, die in den Gemischen aus Säureanhydrid und Carbonat enthalten sein können, seien genannt: Maleinsäureanhydrid und Phthalsäureanhydrid.

Le A 21 434

Als Carbonate, die in den Gemischen aus Säure bzw. Säureanhydrid und Carbonat enthalten sein können, kommen natürliche und synthetische Carbonate und Hydrogencarbonate in Betracht. Speziell genannt seien Alkali- und Erdalkalicarbonate bzw. -Hydrogencarbonate, wie Natriumhydrogencarbonat, Kaliumcarbonat, Calciumcarbonat, Calcit, Aragonit, Kalkstein, Kreide, Marmor, Magnesiumcarbonat, Bittersalz, Kalkspat, Calciumcarbonat-Magnesiumcarbonat-Gemisch, Perlspat, Braunspat, Berylliumcarbonat, Strontiumcarbonat, und ferner Eisen-II-carbonat, Eisenspat, Siderit, Mangancarbonat, Manganspat, Zinkspat, Zinkcarbonat und Galmei.

Als Beispiele für Produkte, die zur Stickstoffabspaltung in der Lage sind, seien genannt: Diazoniumsalze, wie zum Beispiel Benzoldiazoniumchlorid.

Als Zusatzstoffe, die in den erfindungsgemäßen Granulaten enthalten sein können, kommen oberflächenaktive Stoffe, Dispergiermittel, Streckmittel, Bindemittel, Konservierungsmittel und Farbstoffe in Betracht.

Hierbei kommen als oberflächenaktive Substanzen vorzugsweise oberflächenaktive Stoffe anionischer, kationischer oder nicht-ionischer Natur, wie sie z.B. beschrieben werden in "Tenside-Textilhilfsmittel-Waschrohstoffe", Band 1, Seiten 571-835, 837-917 und 963-1041 (1964) sowie in "Grenzflächenaktive Ethylenoxid-Addukte", Seiten 42-95 (1976). Speziell genannt seien Ligninsulfonat, Methylcellulose, Polyoxyethylen-Fettsäureester, Polyoxyethylen-Fettalkoholether, z.B.

Le A 21 434

Alkylarylpolyglykolether, Alkylsulfonate und Eiweißhydrolysate.

Als Dispergiermittel kommen vorzugsweise in Frage:
Kondensationsprodukte aus aromatischen Sulfonsäuren und
Formaldehyd, wie Kondensationsprodukte aus sulfoniertem
Ditolylether und Formaldehyd, ferner Ligninsulfonsäure-
Salze, wie Lithium-, Natrium-, Kalium-, Magnesium-, Calcium- und Ammonium-Salze der Ligninsulfonsäure.

Als Streckmittel kommen alle üblicherweise in wasserdispergierbaren bzw. wasserlöslichen Granulaten verwendbare
Füll- und Trägerstoffe in Betracht. Vorzugsweise verwendbare derartige Stoffe sind anorganische Salze, wie Alkalimetall-, Magnesium- und Ammoniumchloride und -sulfate, z.B. Magnesiumsulfat, Kaliumsulfat, Natriumsulfat,
Kaliumchlorid, Ammoniumsulfat, Lithiumsulfat und Ammoniumchlorid, Silikate, wie Talkum, Kreide, Quarzmehl,
Kaolin, Montmorillonit, Bentonit und Sepiolith, außerdem Graphit, ferner Harnstoff und Harnstoff-Derivate,
wie Hexamethylentetramin und Casein, weiterhin Kohlehydrate, wie Stärke, Zucker, Alginate und deren Derivate,
Getreidemehle, wie Weizenmehl und Reismehl, außerdem
Kelzane, Methylcellulose und Hydroxypropyl-methylcellulose, sowie schließlich wasserlösliche Polymere, wie
Polyvinylalkohol und Polyvinylpyrrolidon.

Als Bindemittel können alle in wasserdispergierbaren
bzw. wasserlöslichen Granulaten üblicherweise vorhandene Bindemittel (Kleber) enthalten sein. Hierzu gehören vorzugsweise Methylcellulose, Zucker, Dextrin, Stärke, Alginate, Glykole, Polyvinylpyrrolidon, Ligninsulfonat, Gummiarabicum, Polyvinylalkohol und Polyvinylacetat.

Beispiele für Konservierungsmittel, die in den erfindungsgemäßen Granulaten enthalten sein können, sind 2-Hydroxybiphenyl, Sorbinsäure, p-Hydroxybenzaldehyd, p-Hydroxybenzoesäuremethylester, Benzaldehyd, Benzoesäure und p-Hydroxybenzoesäurepropylester. Als Farbstoffe, die als Zusatzstoffe in Betracht kommen, seien anorganische Pigmente, wie Eisenoxid, Titandioxid und Ferrocyanblau, und organische Farbstoffe, wie Alizarin-, Azo- und Metallphthalocyaninfarbstoffe genannt.

Als Granulierflüssigkeiten können in den erfindungsgemäßen Granulaten Wasser, organische Lösungsmittel oder Gemische aus Wasser und organischen Lösungsmitteln enthalten sein. Hierbei kommen als organische Lösungsmittel vorzugsweise Alkohole, wie Ethanol und Glycol, Halogenkohlenwasserstoffe, wie Methylenchlorid, und Ether, wie Dioxan und Tetrahydrofuran in Betracht. Besonders bevorzugt ist Wasser als Granulierflüssigkeit.

In den erfindungsgemäßen Granulaten können die prozentualen Anteile der enthaltenen Komponenten innerhalb größerer Bereiche variiert werden. Der Anteil an aktiver Komponente bzw. an aktiven Komponenten liegt im allgemeinen zwischen 5 und 80 Gew.-%, vorzugsweise zwischen 10 und 70 Gew.-%. Der Anteil an zur Gaserzeugung befähigtem Material beträgt im allgemeinen 5 bis 30 Gew.-%, vorzugsweise 10 bis 28 Gew.-%. Die Zusatzstoffe sind im allgemeinen in Anteilen zwischen 2 und 60 Gew.-%, vorzugsweise zwischen 5 und 50 Gew.-% enthalten, und der Anteil an Granulierflüssigkeit liegt im allgemeinen zwischen 1 und 20 Gew.-%, vorzugsweise zwischen 3 und 8 Gew.-%.

Bei der Herstellung der erfindungsgemäßen Granulate können vorzugsweise alle diejenigen Komponenten verwendet werden, die bereits im Zusammenhang mit der Beschreibung der erfindungsgemäßen Granulate vorzugsweise genannt wurden.

Verwendet man bei dem erfindungsgemäßen Verfahren eine oder mehrere aktive Komponenten, die bei Raumtemperatur flüssig sind, so ist es erforderlich, diese Komponenten vor der Granulierung auf feste Trägerstoffe aufzuziehen.

Die Temperaturen können bei dem erfindungsgemäßen Verfahren innerhalb eines größeren Bereiches variiert werden. Im allgemeinen arbeitet man bei Temperaturen zwischen 10 und 80°C, vorzugsweise zwischen 20 und 60°C.

Das erfindungsgemäße Verfahren wird im allgemeinen bei Normaldruck durchgeführt. Es ist jedoch auch möglich, unter erhöhtem oder vermindertem Druck zu arbeiten.

Bei der Durchführung des erfindungsgemäßen Verfahrens geht man im allgemeinen so vor, daß man zunächst mindestens eine aktive Komponente mit mindestens einem zur Gaserzeugung befähigten Produkt sowie mit Zusatzstoffen in dem gewünschten Gewichtsverhältnis vermengt, dieses pulvrige Gemisch fein mahlt, danach durch Zugabe von 1 bis 20 Gew.-%, vorzugsweise 2 bis 10 Gew.-%, bezogen auf das entstehende Produkt, an Granulierflüssigkeit in einem Mischer granuliert, dieses Produkt gegebenenfalls trocknet und anschließend pelletiert oder tablettiert.

Das erfindungsgemäße Verfahren kann sowohl kontinuierlich als auch diskontinuierlich durchgeführt werden.

Zur Durchführung des Mahlvorganges werden bei dem erfindungsgemäßen Verfahren übliche Trockenzerkleinerungsgeräte verwendet. Vorzugsweise in Frage kommen Kugelmühlen, Rotor-Stator-Mühlen, Stiftmühlen, Hammermühlen und Strahlenmühlen, gegebenenfalls mit geeigneten nachgeschalteten Sichtern.

Die Behandlung des pulvrigen Materials mit der Granulierflüssigkeit kann in allen üblichen Mischgeräten vorgenommen werden. Zur Trocknung, die nicht unbedingt erforderlich ist, kommen alle bei der Herstellung von Granulaten üblichen Trockner in Frage. Die abschließende eigentliche Granulatherstellung erfolgt in üblichen Tablettier- bzw. Pelletiermaschinen.

Bei dem erfindungsgemäßen Verfahren erhält man kompakte, staubarme Granulate hoher Dichte mit weitestgehend ein-

Le A 21 434

heitlicher Form und Größe. Gegenüber herkömmlich hergestellten Granulaten zeichnen sich die erfindungsgemäßen Granulate durch verbesserte Dispergierbarkeit bzw. Löslichkeit in Wasser aus.

Die erfindungsgemäßen Granulate lassen sich je nach den enthaltenen aktiven Komponenten für verschiedenste Zwecke einsetzen. Diejenigen Granulate, die agrochemische Wirkstoffe als aktive Komponenten enthalten, lassen sich nach üblichen Methoden im Pflanzenschutz verwenden. Beispielsweise werden derartige Granulate in Wasser dispergiert oder gelöst. Die dabei entstehenden Dispersionen bzw. Lösungen lassen sich gegebenenfalls nach vorherigem Verdünnen nach üblichen Methoden auf die Pflanzen und/oder deren Lebensraum ausbringen, also z.B. durch Spritzen, Sprühen oder Gießen. Die Aufwandmenge richtet sich dabei nach der Konzentration der Dispersion bzw. Lösung und nach der jeweiligen Indikation sowie nach den enthaltenen aktiven Komponenten.

Sind in den erfindungsgemäßen Granulaten keine agrochemischen Wirkstoffe, sondern andere aktive Komponenten enthalten, so erfolgt die Anwendung nach den auf dem jeweiligen Gebiet der Technik üblichen Methoden. Die Aufwandmenge ist auch hier abhängig von den jeweiligen aktiven Komponenten und der jeweiligen Indikation.

Die Herstellung der erfindungsgemäßen Granulate und deren gegenüber vorbekannten Granulaten verbesserte Dispergierbarkeit in Wasser geht aus den nachfolgenden Beispielen hervor.

Le A 21 434

- 15 -

## Herstellungsbeispiele

### Vergleichsbeispiel A

70 Gew.-Teile 4-Amino-3-methyl-6-phenyl-1,2,4-triazin-5(4H)-on werden mit 4 Gew.-Teilen Alkylaryl-sulfonat, 6 Gew.-Teilen Kaolin und 10 Gew.-Teilen Sulfitablauge intensiv gemischt und mit einer mechanischen Mühle gemahlen. Nach Zusatz von 6 Gew.-Teilen Wasser und gutem Vermischen wird die Vormischung mit einer Pelletiermaschine zu einem Aufbaugranulat geformt. Man erhält auf diese Weise Agglomerate, die sich nicht gut in Wasser dispergieren lassen.

### Erfindungsgemäße Beispiele

### Beispiel 1

70 Gew.-Teile 4-Amino-3-methyl-6-phenyl-1,2,4-triazin-5(4H)-on werden mit 4 Gew.-Teilen Alkylaryl-sulfonat, 6 Gew.-Teilen Kaolin, 5 Gew.-Teilen Adipinsäure, 5 Gew.-Teilen Natriumhydrogencarbonat und 10 Gew.-Teilen Sulfitablauge intensiv gemischt und mit einer mechanischen Mühle gemahlen. Nach Zusatz von 6 Gew.-Teilen Wasser und gutem Vermischen wird die Vormischung mit einer Pelletiermaschine zu einem Aufbaugranulat geformt. Man erhält auf diese Weise ein staubfreies und rieselfähiges Produkt, das sich in Wasser gut dispergieren läßt.

Le A 21 434

Beispiel 2

70 Gew.-Teile 4-Amino-3-methyl-6-phenyl-1,2,4-triazin-5(4H)-on werden mit 5 Gew.-Teilen Adipinsäure, 5 Gew.-Teilen Natriumhydrogencarbonat und 20 Gew.-Teilen Sulfitablauge intensiv gemischt und mit einer Strahlenmühle gemahlen. Nach Zusatz von 7 Gew.-Teilen Wasser und gutem Vermischen wird die Vormischung mit einer Pelletiermaschine zu einem Aufbaugranulat geformt, in dem die Teilchen einen Durchmesser von 2 mm und eine Länge von 3 mm aufweisen. Man erhält auf diese Weise ein Produkt, das in Wasser leicht dispergierbar ist. Die entstehende Dispersion läßt sich ohne Schwierigkeiten mit einer Spritze einwandfrei ausbringen.

Beispiel 3

70 Gew.-Teile 4-Amino-3-methyl-6-phenyl-1,2,4-triazin-5(4H)-on werden mit 5 Gew.-Teilen Zitronensäure, 5 Gew.-Teilen Dolomit und 20 Gew.-Teilen Sulfitablauge intensiv gemischt und mit einer mechanischen Mühle gemahlen. Nach Zusatz von 6 Gew.-Teilen Wasser und gutem Vermischen wird die Vormischung mit einer Pelletiermaschine zu einem Aufbaugranulat geformt. Man erhält auf diese Weise ein staubfreies und rieselfähiges Produkt, das sich in Wasser gut dispergieren läßt.

Beispiel 4

70 Gew.-Teile 4-Amino-3-methyl-6-phenyl-1,2,4-triazin-5(4H)-on werden mit 5 Gew.-Teilen Zitronensäure,

Le A 21 434

5 Gew.-Teilen Dolomit und 20 Gew.-Teilen Alkylarylsulfonat intensiv gemischt und mit einer mechanischen Mühle gemahlen. Nach Zusatz von 6 Gew.-Teilen Wasser und gutem Vermischen wird die Vormischung mit einer Pelletiermaschine zu einem Aufbaugranulat geformt. Man erhält auf diese Weise ein staubfreies und rieselfähiges Produkt, das sich in Wasser gut dispergieren läßt.

Beispiel 5

70 Gew.-Teile 4-Amino-6-tert.-butyl-3-methylthio-1,2,4-triazin-5(4H)-on werden mit 6 Gew.-Teilen Kaolin, 5 Gew.-Teilen Adipinsäure, 5 Gew.-Teilen Natriumhydrogencarbonat und 14 Gew.-Teilen Diphenylether-Sulfonsäure-Formaldehyd-Kondensat intensiv gemisch und mit einer mechanischen Mühle gemahlen. Nach Zusatz von 8 Gew.-Teilen Wasser und gutem Vermischen wird die Vormischung mit einer Pelletiermaschine zu einem Aufbaugranulat geformt. Man erhält auf diese Weise ein staubfreies und rieselfähiges Produkt, das sich in Wasser gut dispergieren läßt.

Beispiel 6

70 Gew.-Teile 4-Amino-6-tert.-butyl-3-methylthio-1,2,4-triazin-5(4H)-on werden mit 10 Gew.-Teilen Adipinsäure, 5 Gew.-Teilen Magnesiumcarbonat und 15 Gew.-Teilen Diphenylether-Sulfonsäure-Formaldehyd-Kondensat intensiv gemischt und mit einer mechanischen Mühle gemahlen. Nach Zusatz von 8 Gew.-Teilen Wasser und gutem Vermischen wird die Vormischung mit einer Pelletierma-

Le A 21 434

schine zu einem Aufbaugranulat geformt. Man erhält auf diese Weise ein staubfreies und rieselfähiges Produkt, das sich in Wasser gut dispergieren läßt.

Beispiel 7

25 Gew.-Teile 2-(Ethylthio-methyl-phenyl)-N-methyl-carbamat werden mit 10 Gew.-Teilen Adipinsäure, 9 Gew.-Teilen Magnesiumcarbonat, 10 Gew.-Teilen Montmorillonit, 31 Gew.-Teilen Kaolin und 15 Gew.-Teilen eines Kondensationsproduktes aus Cyclohexanon, Formaldehyd und Natriumbisulfit intensiv gemischt und mit einer mechanischen Mühle gemahlen. Nach Zusatz von 8 Gew.-Teilen Wasser und gutem Vermischen wird die Vormischung mit einer Pelletiermaschine zu einem Aufbaugranulat geformt. Man erhält auf diese Weise ein staubfreies und rieselfähiges Produkt, das sich in Wasser gut dispergieren läßt.

Beispiel 8

10 Gew.-Teile 2-(Ethylthio-methyl-phenyl)-N-methyl-carbamat werden mit 10 Gew.-Teilen Adipinsäure, 9 Gew.-Teilen Magnesiumcarbonat, 10 Gew.-Teilen Montmorillonit, 31 Gew.-Teilen Kaolin und 15 Gew.-Teilen eines Kondensationsproduktes aus Cyclohexanon, Formaldehyd und Natriumbisulfit intensiv gemischt und mit einer mechanischen Mühle gemahlen. Nach Zusatz von 8 Gew.-Teilen Wasser und gutem Vermischen wird die Vormischung mit einer Pelletiermaschine zu einem Aufbaugranulat geformt. Man erhält auf diese Weise ein staubfreies und

Le A 21 434

rieselfähiges Produkt, das sich in Wasser gut dispergieren läßt.

Beispiel 9

25 Gew.-Teile ß-[(1,1'-Biphenyl)-4-yloxy]-α-(1,1-dimethylethyl)-1H-1,2,4-triazol-1-ethanol werden mit 3 Gew.-Teilen Netzmittel auf Basis von Alkylarylsulfonat, 20 Gew.-Teilen Diphenylether-Sulfonsäure-Formaldehydkondensat, 15 Gew.-Teilen Adipinsäure, 7 Gew.-Teilen Natriumhydrogencarbonat und 30 Gew.-Teilen Silitharz (inert) intensiv gemischt und mit einer mechanischen Mühle gemahlen. Nach Zusatz von 6 Gew.-Teilen Wasser und gutem Vermischen wird die Vormischung mit einer Pelletiermaschine zu einem Aufbaugranulat geformt. Man erhält auf diese Weise ein staubfreies und rieselfähiges Produkt, das sich in Wasser gut dispergieren läßt.

Beispiel 10

25 Gew.-Teile ß-[(1,1'-Biphenyl)-4-yloxy]-α-(1,1-dimethyl)-1H-1,2,4-triazol-1-ethanol werden mit 20 Gew.-Teilen Diphenylether-Sulfonsäure-Formaldehydkondensat, 15 Gew.-Teilen Adipinsäure, 7 Gew.-Teilen Natriumhydrogencarbonat und 33 Gew.-Teilen Silitharz (inert) intensiv gemischt und mit einer mechanischen Mühle gemahlen. Nach Zusatz von 6 Gew.-Teilen Wasser und gutem Vermischen wird die Vormischung mit einer Pelletiermaschine zu einem Aufbaugranulat geformt. Man erhält auf diese Weise ein staubfreies

Le A 21 434

und rieselfähiges Produkt, das sich in Wasser gut dispergieren läßt.

Beispiel 11

25 Gew.-Teile ß-/(1,1'-Biphenyl)-4-yloxy_7-ɑ-(1,1-dimethyl)-1H-1,2,4-triazol-1-ethanol werden mit 20 Gew.-Teilen Dispergiermittel auf Basis von Ligninsulfonat, 15 Gew.-Teilen Adipinsäure, 7 Gew.-Teilen Natriumhydrogencarbonat und 33 Gew.-Teilen Silitharz (inert) intensiv gemischt und mit einer mechanischen Mühle gemahlen. Nach Zusatz von 6 Gew.-Teilen Wasser und gutem Vermischen wird die Vormischung mit einer Pelletiermaschine zu einem Aufbaugranulat geformt. Man erhält auf diese Weise ein staubfreies und rieselfähiges Produkt, das sich in Wasser gut dispergieren läßt.

Beispiel 12

50 Gew.-Teile Acetyl-salicylsäure werden mit 5 Gew.-Teilen Zitronensäure, 5 Gew.-Teilen Natriumhydrogencarbonat und 40 Gew.-Teilen Milchzucker intensiv gemischt und mit einer mechanischen Mühle gemahlen. Nach Zusatz von 6 Gew.-Teilen Wasser und gutem Vermischen wird die Vormischung mit einer Pelletiermaschine zu einem Aufbaugranulat geformt. Man erhält auf diese Weise ein staubfreies und rieselfähiges Produkt, das sich in Wasser gut dispergieren läßt.

Le A 21 434

Beispiel 13

50 Gew.-Teile Acetyl-salicylsäure werden mit 5 Gew.-Teilen Zitronensäure, 5 Gew.-Teilen Natriumhydrogencarbonat und 40 Gew.-Teilen Reisstärke intensiv gemischt und mit einer mechanischen Mühle gemahlen. Nach Zusatz von 6 Gew.-Teilen Wasser und gutem Vermischen wird die Vormischung mit einer Pelletiermaschine zu einem Aufbaugranulat geformt. Man erhält auf diese Weise ein staubfreies und rieselfähiges Produkt, das sich in Wasser gut dispergieren läßt.

Beispiel 14

50 Gew.-Teile Acetyl-salicylsäure werden mit 5 Gew.-Teilen Zitronensäure, 5 Gew.-Teilen Natriumhydrogencarbonat, 40 Gew.-Teilen Milchzucker und 5 Gew.-Teilen Vitamin C intensiv gemischt und mit einer mechanischen Mühle gemahlen. Nach Zusatz von 6 Gew.-Teilen Wasser und gutem Vermischen wird die Vormischung mit einer Pelletiermaschine zu einem Aufbaugranulat geformt. Man erhält auf diese Weise ein staubfreies und rieselfähiges Produkt, das sich in Wasser gut dispergieren läßt.

Beispiel 15

80 Gew.-Teile des öllöslichen Farbstoffes Ceresrot G werden mit 5 Gew.-Teilen Adipinsäure, 5 Gew.-Teilen Natriumhydrogencarbonat und 10 Gew.-Teilen Sulfitablauge intensiv gemischt und mit einer mechanischen

Le A 21 434

Mühle gemahlen. Nach Zusatz von 6 Gew.-Teilen Wasser und gutem Vermischen wird die Vormischung mit einer Pelletiermaschine zu einem Aufbaugranulat geformt. Man erhält auf diese Weise ein staubfreies und rieselfähiges Produkt, das sich in Wasser gut dispergieren läßt.

Beispiel 16

70 Gew.-Teile eines wasserlöslichen Azofarbstoffs werden mit 5 Gew.-Teilen Adipinsäure, 5 Gew.-Teilen Natriumhydrogencarbonat und 20 Gew.-Teilen Sulfitablauge intensiv gemischt und mit einer mechanischen Mühle gemahlen. Nach Zusatz von 6 Gew.-Teilen Wasser und gutem Vermischen wird die Vormischung mit einer Pelletiermaschine zu einem Aufbaugranulat geformt. Man erhält auf diese Weise ein staubfreies und rieselfähiges Produkt, das sich in Wasser gut dispergieren läßt.

Beispiel 17

5 Gew.-Teile 1-(4-Chlorphenoxy)-3,3-dimethyl-1-(1,2,4-triazol-1-yl)-2-butanon werden mit 20 Gew.-Teilen Sulfitablauge, 10 Gew.-Teilen Bernsteinsäure, 5 Gew.-Teilen Magnesiumcarbonat, 10 Gew.-Teilen Montmorillonit und 50 Gew.-Teilen Kaolin intensiv vermischt und mit einer mechanischen Mühle gemahlen. Nach Zusatz von 7 Gew.-Teilen Wasser und gutem Vermischen wird die Vormischung mit einer Tablettiermaschine zu einem Aufbaugranulat geformt. Man erhält auf diese Weise ein staubfreies und rieselfähiges Produkt, das sich in Wasser gut dispergieren läßt.

Le A 21 434

Beispiel 18

25 Gew.-Teile 1-(4-Chlorphenoxy)-3,3-dimethyl-1-(1,2,4-triazol-1-yl)-2-butanon werden mit 20 Gew.-Teilen Sulfitablauge, 10 Gew.-Teilen Bernsteinsäure, 5 Gew.-Teilen Magnesiumcarbonat, 10 Gew.-Teilen Montmorillonit und 30 Gew.-Teilen Kaolin intensiv vermischt und mit einer mechanischen Mühle gemahlen. Nach Zusatz von 7 Gew.-Teilen Wasser und gutem Vermischen wird die Vormischung mit einer Tablettiermaschine zu einem Aufbaugranulat geformt. Man erhält auf diese Weise ein staubfreies und rieselfähiges Produkt, das sich in Wasser gut dispergieren läßt.

Beispiel 19

5 Gew.-Teile 1-(4-Chlorphenoxy)-3,3-dimethyl-1-(1,2,4-triazol-1-yl)-2-butanon werden mit 20 Gew.-Teilen Sulfitablauge, 10 Gew.-Teilen Itaconsäure, 5 Gew.-Teilen Magnesiumcarbonat, 10 Gew.-Teilen Montmorillonit und 50 Gew.-Teilen Kaolin intensiv vermischt und mit einer mechanischen Mühle gemahlen. Nach Zusatz von 7 Gew.-Teilen Wasser und gutem Vermischen wird die Vormischung mit einer Tablettiermaschine zu einem Aufbaugranulat geformt. Man erhält auf diese Weise ein staubfreies und rieselfähiges Produkt, das sich in Wasser gut dispergieren läßt.

Beispiel 20

5 Gew.-Teile 1-(4-Chlorphenoxy)-3,3-dimethyl-1-(1,2,4-

Le A 21 434

triazol-1-yl)-2-butanon werden mit 20 Gew.-Teilen Sulfitablauge, 10 Gew.-Teilen Bernsteinsäure, 5 Gew.-Teilen Magnesiumcarbonat, 10 Gew.-Teilen Montmorillonit und 50 Gew.-Teilen Kaolin intensiv vermischt und mit einer mechanischen Mühle gemahlen. Nach Zusatz von 7 Gew.-Teilen Wasser und gutem Vermischen wird die Vormischung mit einer Tablettiermaschine zu einem Aufbaugranulat geformt. Man erhält auf diese Weise ein staubfreies und rieselfähiges Produkt, das sich in Wasser gut dispergieren läßt.

Patentansprüche

1) Wasserdispergierbare bzw. wasserlösliche Granulate, gekennzeichnet durch einen Gehalt an

- 5 bis 80 Gew.-% an mindestens einer aktiven Komponente,
- 5 bis 30 Gew.-% an mindestens einem zur Gaserzeugung befähigten Produkt,
- 2 bis 60 Gew.-% an Zusatzstoffen und gegebenenfalls
- 1 bis 20 Gew.-% an Granulierflüssigkeit,

wobei die Summe der Bestandteile jeweils 100 Gew.-% beträgt.

2) Granulate gemäß Anspruch 1, dadurch gekennzeichnet, daß als aktive Komponente mindestens ein Stoff aus der Gruppe der agrochemischen Wirkstoffe, der Wirkstoffe zur Bekämpfung von Schädlingen im Haushalts- und Hygienebereich, der pharmakologisch wirksamen Stoffe oder der Farbstoffe enthalten ist.

3) Granulate gemäß Anspruch 1, dadurch gekennzeichnet, daß als zur Gaserzeugung befähigtes Produkt mindestens ein Gemisch aus Säure und Carbonat, ein Gemisch aus Säureanhydrid und Carbonat, eine Stickstoff abspaltende Substanz und/oder mindestens ein aus Bakterien und Milchsäure bestehendes Gemisch, das zur Kohlendioxid-Erzeugung in der Lage ist, enthalten ist.

Le A 21 434

4) Granulate gemäß Anspruch 1, dadurch gekennzeichnet, daß als Zusatzstoffe oberflächenaktive Stoffe, Dispergiermittel, Streckmittel, Bindemittel, Konservierungsmittel und/oder Farbstoffe enthalten sind.

5) Granulate gemäß Anspruch 1, dadurch gekennzeichnet, daß als Granulierflüssigkeit Wasser, mindestens ein organisches Lösungsmittel oder ein Gemisch aus Wasser und mindestens einem organischen Lösungsmittel enthalten ist.

6) Verfahren zur Herstellung von in Wasser dispergierbaren bzw. löslichen Granulaten, dadurch gekennzeichnet, daß man

- mindestens eine aktive Komponente mit mindestens einem zur Gaserzeugung befähigten Produkt sowie mit Zusatzstoffen vermengt,
- das entstehende Gemisch fein mahlt, danach mit Granulierflüssigkeit versetzt, gegebenenfalls trocknet und
- pelletiert oder tablettiert.

7) Verwendung von Granulaten gemäß Anspruch 1 in der Landwirtschaft und im Gartenbau, im Haushalts- und Hygienebereich, im medizinischen Bereich oder in der Farbstoffindustrie.

Le A 21 434

8. Granulate gemäß Anspruch 1, dadurch gekennzeichnet, daß als aktive Komponente 4-Amino-3-methyl-6-phenyl-1,2,4-triazin-5(4H)-on enthalten ist.

9. Granulate gemäß Anspruch 1, dadurch gekennzeichnet, daß als aktive Komponente 2-(Ethylthio-methyl-phenyl)-N-methyl-carbamat enthalten ist.

10. Granulate gemäß Anspruch 1, dadurch gekennzeichnet, daß als aktive Komponente ß-/(1,1'-Biphenyl)-4-yloxy/-X-(1,1-dimethylethyl)-1H-1,2,4-triazol-1-ethanol enthalten ist.